# EUROPÄISCHE PATENTANMELDUNG

(11) **EP 1 637 123 A1**
(43) Veröffentlichungstag der Anmeldung: **22.03.2006**
(21) Anmeldenummer: 05017724.5
(22) Anmeldetag: 16.08.2005
(51) Int. Cl.: A61K 8/66, A61K 8/41, A61K 8/35, A61Q 5/00, A61Q 5/12

(54) **Zubereitung mit enzymatisch katalysierter Freisetzung von Pflegestoffen für keratinische Fasern in situ**

(30) Priorität: 26.08.2004 DE 102004041568
(71) Anmelder: Henkel Kommanditgesellschaft auf Aktien, 40589 Düsseldorf (DE)
(72) Erfinder: Kleen, Astrid, 22763 Hamburg (DE); Höffkes, Horst, 40595 Düsseldorf (DE); Brabänder, Oliver, 46049 Oberhausen (DE)

(57) **Zusammenfassung**

Die vorliegende Erfindung betrifft eine Zubereitung mit enzymatisch katalysierter Freisetzung von Pflegestoffen für keratinische Fasern in situ.

## Beschreibung

Die vorliegende Erfindung betrifft eine Zubereitung mit enzymatisch katalysierter Freisetzung von Pflegestoffen für keratinische Fasern in situ.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Dieses Verhalten führt dazu, daß Haare in vielfältiger Weise strapazierenden Einflüssen ausgesetzt sind, die sich negativ auf die Oberflächenstruktur auswirken.

Es hat daher nicht an Anstrengungen gefehlt, der Schädigung der Haarstruktur durch Einsatz von Pflegewirkstoffen entgegenzuwirken bzw. vorzubeugen. Es wurden vielfältige Pflegekomponenten entwickelt, die gezielt als Nachbehandlungsmittel für geschädigtes Haar zum Einsatz kommen. Ferner wurden die Haarbehandlungsmittel an sich, wie beispielsweise die Fixiermittel im Rahmen einer Dauerwellbehandlung oder die Färbemittel, mit zusätzlichen Pflegestoffen versetzt. So wurde beispielsweise in der DE-A1-196 17 569 vorgeschlagen, spezielle Aminosäuren als Pflegestoffe zu verwenden.

Aus der DE-A-0019945487 ist ein Verfahren zur Restrukturierung keratinischer Fasern bekannt, bei dem auf Fasern mindestens ein Enzym vom Typ der Carboxylesterhydrolasen und mindestens ein Wirkstoff, der eine Substrataktivität für das Enzym aufweist, aufgebracht werden.

Die EP-A-0 713 696 beschreibt ein Lipase enthaltendes Produkt zur topischen Anwendung, das befähigt sein soll, auf der Haut eine Hydroxysäure freizusetzen und seine Verwendung zur sanften Behandlung der Haut, einschließlich der Kopfhaut.

Aus der EP-A-0 530 865 ist eine Lipase enthaltende Zusammensetzung zur Reinigung von Haut, Kopfhaut und Haaren bekannt.

Die DE-C-197 09 334 offenbart den Einsatz proteolytisch aktiver Enzyme zur enzymatischen Auflösung oder Ablösung der eine unerwünschte Rauhigkeit der Haaroberfläche verursachenden abgespreizten Kutikularänder des Haares.

Überraschenderweise wurde nun gefunden, daß Enzyme, vorzugsweise Oxidoreduktasen und insbesondere solche der Klassen EC 1.5.3.1, EC 1.5.3.2 und EC 1.5.3.4 gemäß der Klassifikation des *Nomenclature Committee of the International Union of Biochemistry and Molecular Biology,* die im Internet unter der URL http://www.chem.qmul.ac.uk/iubmb/enzyme/ recherchierbar ist, eingesetzt werden können, um Pflegewirkstoffe auf die Oberfläche von keratinischen Fasern, vorzugsweise Haaren, aufzubringen.

Gegenstand der vorliegenden Erfindung ist somit ein Verfahren zur enzymatischen Freisetzung von Pflegestoffen für keratinische Fasern in situ, das dadurch gekennzeichnet ist, daß man
a) ein Substrat A, das befähigt ist, bei enzymatischer Umsetzung Pflegestoffe für keratinische Fasern freizusetzen und
b) ein zur Umsetzung des Substrates A befähigtes Enzym B
nacheinander oder gemeinsam auf die zu behandelnde keratinische Faser aufbringt und so Pflegestoffe C und/oder D in situ erzeugt.

Geeignete Substrate A sind insbesondere Sarkosin, L-Aminosäuren, N-Methyl-L-Leucin, N-Methyl-D/L-Valin, N-Methyl-D/L-Alanin und *N*⁶-methyl-L-Lysin.

Geeignete Enzyme B sind vorzugsweise Oxidoreduktasen und insbesondere solche der Klassen EC 1.5.3.1 (Sarcosin Oxidase), EC 1.5.3.2 (N-methyl-L-amino-acid Oxidase) und EC 1.5.3.4 (*N*⁶-methyl-lysine Oxidase). Eine bevorzugt einsetzbare Sarcosin Oxidase ist beispielsweise die aus rekombinanten E. coli Zellen von der Firma Roche erhältliche und unter der URL http://www.roche-applied-science.com/indbio_neu/industry/clinical/pdf/enzymes/ S098-100.pdf beschriebene Sarcosin Oxidase.

Pflegestoffe C sind insbesondere Aminosäuren wie L-Leucin, L-Lysin, L-Valin und Glycin; Pflegestoffe D sind vorzugsweise Formaldehyd und Acetaldehyd.

Enzymatische Umsetzungen gemäß des erfindungsgemäßen Verfahrens sind beispielsweise

| | |
|---|---|
| Sarcosin | → Glycin + Formaldehyd, |
| N-Methyl-L-leucin | → L-Leucin + Formaldehyd, |
| N-Methyl-D/L-alanin | → L-Valin + Formaldehyd, oder |
| N-Methyl-D/L-valin | → Glycin + Acetaldehyd. |

Ein weiterer Gegenstand der vorliegenden Erfindung ist eine Zubereitung mit enzymatisch katalysierter Freisetzung von Pflegestoffen für keratinische Fasern in situ, die ein
Substrat A enthält, das befähigt ist, bei enzymatischer Umsetzung Pflegestoffe für keratinische Fasern freizusetzen und ein
zur Umsetzung des Substrates A befähigtes Enzym B enthält, das dadurch gekennzeichnet ist, daß
bei Umsetzung des Substrates A auf der zu behandelnden keratinischen Faser durch das Enzym B Pflegestoffe C und/oder D in situ erzeugt werden.

Da die erfindungsgemäße enzymatische Umsetzung Luftsauerstoff erfordert, können A und B unter Luftabschluß in einer Zubereitung vorliegen, ohne daß es zu einer verfrühten Umsetzung kommt.

Von der Erfindung umfasst ist jedoch auch ein 2-Komponenten-System, bei dem Substrat und Enzym nacheinander auf die keratinische Faser aufgebracht werden.

Ein weiterer Gegenstand der vorliegenden Erfindung ist ein zweiteiliges Kit zur enzymatisch katalysierten Freisetzung von Pflegestoffen C und/oder D für keratinische Fasern in situ, dadurch gekennzeichnet, daß es
a) Substrate A und
b) Enzyme B
umfasst.

Ein weiterer Gegenstand der vorliegenden Erfindung ist die Verwendung von Substraten A und Enzymen B zur enzymatisch katalysierten Freisetzung von Pflegestoffen C und/oder D für keratinische Fasern in situ.

Unter Pflegestoffen bzw. Pflegewirkstoffen im Sinne der Erfindung sind solche Wirkstoffe zu verstehen, die den durch verschiedenartigste Einflüsse entstehenden Schädigungen keratinischer Fasern entgegenwirken bzw. diesen Schädigungen vorbeugen.
Hierbei spielt beispielsweise die Wiederherstellung der natürlichen Festigkeit eine wesentliche Rolle. Beispiele für schädigende Einflüsse sind Dauerwellbehandlungen, oxidative Färbungen oder Aufhellungen der Haare sowie häufiges Waschen, Fönen und Kämmen. Weiterhin können Schädigungen durch Umwelteinflüsse, wie beispielsweise UV-Licht, auftreten.

Erfindungsgemäß ausgerüstete Fasern zeichnen sich beispielsweise durch einen verbesserten Glanz, durch einen verbesserten Griff und durch eine leichtere Kämmbarkeit aus. Ferner läßt sich eine erfolgreiche Ausrüstung physikalisch als Schmelzpunktserhöhung im Vergleich zur geschädigten Faser nachweisen.

Das erfindungsgemäße Verfahren führt außerdem zur Wiederherstellung der natürlichen Hydrophobie der Haaroberfläche. Mit zunehmender Haarschädigung wird die Haaroberfläche zunehmend hydrophiler. Durch die hier beschriebene Behandlung erhält das Haar seine wasserabweisende Oberflächenausrüstung zurück (Imprägnierung).

Neben der beschriebenen Wirkung auf geschädigte Fasern ist ein zweiter Aspekt der vorliegenden Erfindung auch die Erzielung einer haarfestigenden Wirkung bei ungeschädigtem Haar. Das erfindungsgemäße Verfahren kann daher auch zur Festigung der Haare für spezielle Stylingeffekte angewendet werden.

Unter keratinischen Fasern werden erfindungsgemäß Pelze, Wolle, Federn und insbesondere menschliche Haare verstanden.

Erfindungsgemäß werden die Pflegestoffe C in situ generiert, was besonders vorteilhaft ist, da die Pflegestoffe so nicht über längere Zeit den oftmals sauren oder gar alkalischen Medien von Haarpflegeprodukten ausgesetzt sind, in denen sie ansonsten inaktiviert werden könnten.

Bevorzugtermaßen wird das erfindungsgemäße Verfahren so durchgeführt, daß man eine bestmögliche Umsetzung von A zu C und/oder D mittels B erhält. Geeignete Bedingungen hierfür können durch den Fachmann in Abhängigkeit von der gewünschten Reaktion eingestellt werden.

Die Komponenten A und B sind in der erfindungsgemäßen Zubereitung bevorzugt in Mengen von 0,001 bis 99 Gew.-%, bezogen auf die gesamte Zubereitung, enthalten. Mengen von 0,01 bis 2 Gew.-% sind besonders bevorzugt.

Das Massenverhältnis des Enzyms B zum Substrat A beträgt bevorzugterweise 1:40000 bis 1:1, besonders bevorzugt ist ein Massenverhältnis von 1:2000 bis 1:50.

Hinsichtlich des zeitlichen Ablaufs des Verfahrens unterliegt die Erfindung keinerlei Beschränkungen. Es ist prinzipiell möglich, zwei separate Zubereitungen, enthaltend (a) das Substrat und (b) das Enzym nacheinander in beliebiger Reihenfolge auf die Fasern aufzubringen. Hierbei sollte allerdings zwischen den Schritten (a) und (b), kein allzu großer zeitlicher Abstand liegen, so daß die Fasern zwischen den Schritten nicht trocknen.

Obwohl diese 2-stufigen Verfahren zu den gewünschten Effekten führen, kann es bevorzugt sein, das erfindungsgemäße Verfahren in einem 1-Schritt-Prozeß durchzuführen, da diese Prozesse einfacher anzuwenden sind. Dabei kann das Substrat A zusammen mit dem Enzym B aufgebracht werden.

Obwohl die Zubereitung prinzipiell auf dem Haar verbleiben kann, wird in einer bevorzugten Ausführungsform des erfindungsgemäßen Verfahrens die Zubereitung nach einer Einwirkzeit von 1 Minute bis 2 Stunden ausgespült. Dieses Ausspülen kann mit reinem Wasser oder einem marktüblichen Shampoo erfolgen. Einwirkzeiten von 5 bis 15 Minuten haben sich in den meisten Fällen als ausreichend erwiesen.

Unabhängig von dem Ablauf des erfindungsgemäßen Verfahrens hat es sich als vorteilhaft erwiesen, die Zubereitung bei einer Temperatur von 20 bis 55 °C, insbesondere von 35 bis 40°C, anzuwenden.

Die Art der Herstellung der erfindungsgemäßen Zubereitung unterliegt keinen prinzipiellen Einschränkungen. Erfindungsgemäß geeignet sind insbesondere wäßrige, alkoholische und ölige Zubereitungen sowie deren Mischungen. Besonders bevorzugt sind wasserarme bzw. wasserfreie Zubereitungen. Es kann sich beispielsweise um Lösungen, Dispersionen, Öle und Emulsionen (Wasser in Öl-Emulsionen, Öl in Wasser-Emulsionen sowie multiple Emulsionen und PIT-Emulsionen) handeln. Der pH-Wert dieser Zubereitungen liegt in der Regel bei 2 bis 10, bevorzugt bei 4 bis 9 und besonders bevorzugt bei 6 bis 8.

In einer bevorzugten Ausführungsform der vorliegenden Erfindung werden die Zubereitungen in Form einer verdickten Lösung formuliert. Zu diesem Zweck werden die Zubereitungen mit Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol oder auch Poylacrylsäurepolymere angedickt. Bevorzugt werden die Zubereitungen niedrigviskos formuliert.

Die Zubereitungen können außer dem Enzym und ggf. dem Pflegewirkstoff mit Substrataktivität alle üblichen Bestandteile enthalten, die für die Behandlung keratinischer Fasern, insbesondere menschlicher Haare, geeignet sind. Bevorzugt sind wasserarme Zubereitungen. Unter wasserarmen Systemen werden Zubereitungen mit einem Gehalt an Wasser kleiner 5 % verstanden.

Es hat sich als vorteilhaft erwiesen, wenn die Zubereitung mindestens ein Tensid enthält. Es kann sich dabei sowohl um anionische, ampholytische, zwitterionische oder nichtionogene Tenside als auch um kationische Tenside handeln. Der Fachmann kann einen eventuellen Einfluß der verschiedenen Tenside auf die Aktivität des Enzyms vom Typ der Carboxylesterhydrolasen gegebenenfalls durch einfache Vorversuche überprüfen.

In einer bevorzugten Ausführungsform der vorliegenden Erfindung wird eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N, N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈₋Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin enthalten die erfindungsgemäß verwendeten Zubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öl- oder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-isopentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl- glycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol und Decadienol, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die erfindungsgemäß verwendeten Zubereitungen enthalten gemäß einer bevorzugten Ausführungsform mindestens einen zusätzlichen Pflegestoff. Dieser Pflegestoff ist bevorzugt ausgewählt aus kationischen Polymeren und Silikonen.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Unter den kationischen Polymeren sind aber die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- Kationische Guar-Derivate, wie insbesondere die unter den Handelsnamen Cosmedia®Guar und Jaguar® vertriebenen Produkte,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
- sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquaternium-32, Polyquaternium-35 und Polyquaternium-37 (Handelsprodukte z. B. Salcare® SC 92 und Salcare®SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer® JR 400, und Polymere vom Typ Polyquaternium-2, insbesondere das Handelsprodukt Mirapol®A-15, sind ganz besonders bevorzugte kationische Polymere.

Die kationischen Polymeren sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignet als Pflegestoff in Kombination mit oder alternativ zu kationischen Polymeren sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO-- oder -SO₃--Gruppen enthalten, und solche Polymeren zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat®2001 N im Handel erhältlich sind, sind erfindungsgemäß besonders bevorzugte Ampho-Polymere.

Erfindungsgemäß verwendbare Pflegestoffe sind weiterhin Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80) und das Handelsprodukt Fancorsil® LIM-1. Ein geeignetes anionisches Silikonöl ist das Produkt Dow Corning®1784.

Neben dem Enzym vom Typ der Carboxylesterhydrolasen und den weiteren, oben genannten bevorzugten Komponenten können die Zubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- Strukturanten wie Maleinsäure, Milchsäure und Aminosäuren
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Panthenol und Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

## Patentansprüche

1. Verfahren zur enzymatischen Freisetzung von Pflegestoffen für keratinische Fasern in situ, das **dadurch gekennzeichnet ist, daß** man
a) ein Substrat A, das befähigt ist, bei enzymatischer Umsetzung Pflegestoffe für keratinische Fasern freizusetzen und
b) ein zur Umsetzung des Substrates A befähigtes Enzym B
nacheinander oder gemeinsam auf die zu behandelnde keratinische Faser aufbringt und so Pflegestoffe C und/oder D in situ erzeugt.

2. Verfahren nach Anspruch 1, **dadurch gekennzeichnet, daß** das Substrat A ausgewählt ist unter Sarkosin, L-Aminosäuren, N-Methyl-L-Leucin, N-Methyl-D/L-Valin, N-Methyl-D/L-Alanin und *N*⁶-methyl-L-Lysin.

3. Verfahren nach Anspruch 1 oder 2, **dadurch gekennzeichnet, daß** das Enzym B ausgewählt ist unter Oxidoreduktasen, insbesondere unter Sarcosin Oxidase, N-methyl-L-amino-acid Oxidase und *N*⁶-methyl-lysine Oxidase.

4. Verfahren nach einem der vorhergehenden Ansprüche, **dadurch gekennzeichnet, daß** die Pflegestoffe C ausgewählt sind unter Aminosäuren, insbesondere unter L-Leucin, L-Lysin, L-Valin und Glycin.

5. Verfahren nach einem der vorhergehenden Ansprüche, **dadurch gekennzeichnet, daß** die Pflegestoffe D ausgewählt sind unter Formaldehyd und Acetaldehyd.

6. Zubereitung mit enzymatisch katalysierter Freisetzung von Pflegestoffen für keratinische Fasern in situ, die ein
Substrat A enthält, das befähigt ist, bei enzymatischer Umsetzung Pflegestoffe für keratinische Fasern freizusetzen und ein
zur Umsetzung des Substrates A befähigtes Enzym B enthält, das **dadurch**
**gekennzeichnet ist, daß**
bei Umsetzung des Substrates A auf der zu behandelnden keratinischen Faser durch das Enzym B Pflegestoffe C und/oder D in situ erzeugt werden.

7. Verwendung von Substraten A und Enzymen B zur enzymatisch katalysierten Freisetzung von Pflegestoffen C und/oder D für keratinische Fasern in situ.

8. Zweiteiliges Kit zur enzymatisch katalysierten Freisetzung von Pflegestoffen C und/oder D für keratinische Fasern in situ, **dadurch gekennzeichnet, daß** es
a) Substrate A und
b) Enzyme B
umfasst.
